# EUROPEAN PATENT SPECIFICATION

(11) **EP 0 289 287 B1**
(45) Date of publication and mention of the grant of the patent: **28.01.1998**
(21) Application number: 88303803.6
(22) Date of filing: 27.04.1988
(51) Int. Cl.: C07K 14/575, C12N 15/12, G01N 33/53, A61K 38/17

(54) **Amyloid peptides**
Amyloid-Peptide
Peptides amyloides

(30) Priority: 27.04.1987 GB 8709871
(43) Date of publication of application: 02.11.1988
(60) Divisional application: 97203242.9
(73) Proprietor: AMYLIN PHARMACEUTICALS, INC., San Diego California 92121 (US)
(72) Inventor: Cooper, Garth James Smith, Woodstock Oxon. OX7 1SR (GB); Willis, Anthony Charles, Witney Oxon. OX7 1SR (GB)
(74) Representative: Baillie, Iain Cameron

(56) References cited:
- EP-A- 0 191 349
- EP-A- 0 263 802
- EP-A- 0 309 100
- PNAS vol. 84, 3881-3885 (1987)
- Biochemical and Biophysical Research Communications, vol 140, no.3, 14.11.1986, pages 827-831.
- PNAS vol.84, p.8628-8632, 1987

## Description

This invention relates to peptides and in a preferred embodiment to a peptide isolatable from the pancreas of diabetics.

The most common form of diabetes is a particular type of diabetes mellitus (designated as Type 2 or non-insullin-dependent diabetes mellitus), which may affect more than 10% of the United States population in their late sixties (Health and Nutrition Examination Survey, Cycle II 1976-80. National Center for Health Statistics). Frequently, an unusual proteinaceous deposit is observed precipitated in the islets of Langerhans in the pancreas of such diabetic patients. The proteinaceous deposit is known as amyloid and has been previously reported (Opie E.L. J. Exper. Med 5:529-40 (1900) and Bell Diabetes 1:341-344 (1952)).

It appears that the accumulation of amyloid particles within the islets of Langerhans is specific for the pancreas of diabetic persons. Within the particles, one component is the peptide (previously tentatively termed "Diabetes Associated Peptide" or "DAP", and now definitively named herein "amylin").

Westermark et al. in Biochemical and Biophysical Research Communications 140 93 827-831 (1986) give a partial description of an impure peptide deposited as amyloid fibrills in an insulin expressing tumour. They additionally state that they have some speculative evidence for its deposition in the islets of Langerhans in Type 2 Diabetes Mellitus. The sequence of the first 19 amino acids of the Westermark et al. peptide is given, but from this partial sequence it appears to be different from the peptides in accordance with the present invention as it has a serine residue at position 7. In addition, it is by no means clear that the residue at position 2 is cysteine.

The peptide analysed by Westermark et al., whatever its structure, appears to be very impure. 1.1 micrograms of material were obtained after the crude HPLC purification carried out, but only 12.2 picomoles of lysine were found after the first cycle of Edman degradation during the amino acid analysis sequence. This implies that only in the order of 30 picomoles of peptide were present in the crude extract of potentially 268 picomoles.

According to a first aspect of the present invention, there is provided a synthetic peptide having the amino acid sequence: 
5    10    15    20    25    30    35

 having a disulphide bridge between the cysteine residues.

The amino acid residues are designated by the usual single letter nomenclature. The more recent single letter designations may be correlated with the classical three letter designations of amino acid residues as follows:

| | | | |
|---|---|---|---|
| D = | Asp | M = | Met |
| E = | Glu | C = | Cys |
| F = | Phe | L = | Leu |
| K = | Lys | I = | Ile |
| N = | Asn | H = | His |
| Q = | Gln | T = | Thr |
| R = | Arg | S = | Ser |
| W = | Trp | P = | Pro |
| Y = | Tyr | G = | Gly |
| | | V = | Val |
| | | A = | Ala |

It is believed that in the natural amylin peptide the cysteine residues at positions 2 and 7 cooperate to form a disulphide linkage.

The invention also provides a peptide having the above amino acid sequence in which the 37th amino acid is tyrosine amide.

The invention also provides a peptide which is a conservative substitution of the above peptide.

The invention also provides a synthetic peptide which is a sub-fragment of the peptide having the amino acid sequence given above including the sequence VGSNTY and having a disulphide bridge between the cysteine residues.

The peptide of the invention can be used in human or animal therapy.

The invention also provides an isolated DNA or RNA sequence coding for the above peptide sequences, and provides a vector comprising the above sequence as well as a host cell comprising such a vector.

Peptides in accordance with the invention may consist only of the above sequence. Alternatively, additional amino acid residues (for example one or two or even a much larger number) may be present.

Peptides which have been post-translationally modified (eg. glycosylated) or similarly modified by other means (especially on the S residue at position 20) are also included within the scope of the invention.

Preferably the peptide of the invention is substantially pure. By "substantially pure" is meant purity in excess of 50%, particularly 80%, for example 90% and especially 95 or 99% by weight.

In another aspect a peptide in accordance with the invention can be used in a process for preparing material for an immunoassay for the confirmation or prediction of Type 2 diabetes mellitus.

The invention also extends to antibodies (eg. monoclonal antibodies) which are selective for the peptides of the invention.

The invention also provides hybridoma cells capable of producing such monoclonal antibodies.

Such antibodies are optionally labelled. Such antibodies can be used for directing a pharmaceutical agent or enzyme against amyloid in the pancreas of a diabetic patient.

A peptide in accordance with another aspect of the invention may be prepared from diabetic pancreata. Accordingly in this aspect the invention provides a method of preparing a peptide, having the above sequence, from diabetic pancreata by a process comprising the steps of:
(a) homogenizing said pancreata;
(b) heating the material resulting from step (a);
(c) digesting the material from step (b) using collagenase;
(d) centrifuging the material from step (c) and collecting the resulting amyloid material;
(e) solubilizing the amyloid material from step (d) using 70% formic acid in conjunction with ultrasound and cooling;
(f) immediately removing the formic acid from the material of step (e);
(g) redissolving the material from step (f) and performing gel filtration chromatography using a mobile phase comprising aqueous guanidine; and
(h) subjecting the material from step (g) to reverse phase high performance liquid chromatography using a mobile phase comprising trifluoroacetic acid and elution by an acetonitrile gradient.

The invention also relates to a peptide obtainable from diabetic pancreata by said process. In particular there is provided a peptide obtainable by the above process which is resistant to carboxy-peptidase Y.

Such a peptide can have vasodilator activity.

Preferably the reverse phase HPLC (in which the stationary phase is hydrophobic) is run immediately after the normal phase HPLC gel filtration; this helps ensure no loss of peptide in storage.

The immediate reverse phase HPLC purification step has a number of other advantages. First of all, it enables a substantially pure peptide to be isolated. Secondly, the effluent from the gel filtration is conveniently desalted. Thirdly, the peptide is concentrated to a smaller volume.

Amyloid may be solubilised in formic acid. Preferably ultra sound is used to effect or help effect solubilisation.

Amyloid is preferably obtained from the pancreas of a diabetic. The pancreas may be digested by a proteolytic enzyme such as collagenase. The pancreas may be prior heated (for example to 70°C) to assist the digestion by the melting of collagen fibrils.

It appears that in the pancreas of diabetics (at least diabetics suffering from Type 2 Diabetes Mellitus), amylin is produced and deposited as amyloid. The level of production appears to be higher than the level of production in the normal (non-diabetic) pancreas. The normal pancreas may also make a peptide with one or two amino acid differences from amylin. Amylin and amylin-like peptides may therefore be useful as a standard and, when detectably labelled, as a probe for use in immunoassays.

The antibodies of the invention can be used as a specific probe for diabetes associated peptide (amylin) having the sequence given above and are useful for immunohistochemical or immunoassay purposes. The antibody might be used in a diagnostic test for patients who have abnormal amounts of amylin, eg. in their plasma. In addition, when the antibody is labelled it might be used in vivo to detect the presence of amylin in amyloids in the pancreas of affected patients. It could also be used to direct a pharmaceutical agent or enzyme, eg. one bound to it, which might be used to disperse the amyloid in the islets, or to inhibit growth of the amyloid. Both types of such antibodies are also preferred features of the present invention. The antibody can optionally be a monoclonal antibody, produced by a hybridoma.

Although the preparation of amylin has been demonstrated by extraction from pancreatic tissue, amylin and amylin-like peptides may be synthesized. They may also be produced by recombinant DNA techniques. The first stage in such techniques would be to obtain a length of DNA coding for amylin or a amylin-like peptide. One way to do this would be to isolate mRNA from amylin-producing cells and, with the in vitro use of reverse transcriptase produce cDNA coding for the amylin or a amylin-like peptide. Oligonucleotide probes can be produced from the known amino acid sequence and can be used to screen cDNA or genomic DNA libraries. Alternatively, given that the nucleotide sequence will generally only be just over 100 bases in length, the DNA may be chemically synthesized. A number of oligonucleotides may be produced, from which the desired cDNA can be prepared by the use of DNA polymerase and DNA ligase. Restriction endonuclease digestion of either end can leave appropriate cohesive restriction sites for insertion into a plasmid.

The genetic sequence of amylin is as listed as follows. This is a preferred embodiment, but the invention also includes conservative substitutions thereof, according to the genetic code.

5'

AAG TGC AAC ACA GCC ACC TGT GCC ACT CAA CGG CTG GCA AAT TTT TTA


GTT CAT TCC AGC AAC AAC TTT GGT GCC ATT CTC TCA TCT ACC AAT GTG

3'

GGT TCC AAT ACC TAT

Whether the synthetic DNA is cDNA or chemically synthesized, it can either have cohesive ends provided by a restriction endonuclease or it may be terminally tailed by for example oligo-dC by the use of the appropriate nucleotide and terminal transferase.

Whichever tailing method is chosen, a plasmid (for example pBR322) can then be taken and cleaved at a single site by a restriction endonuclease such as PstI. PstI cleaves pBR322 in the gene coding for ampicillin resistance. This allows for easy selection of recombinant plasmids. If desired, the PstI digested pBR322 can be oligo-dG tailed to complement an oligo-dC tailed piece of DNA coding for amylin or a amylin-like peptide. The cleaved plasmid and the DNA coding for amylin or a amylin-like peptide can be annealed and ligated and host cells (for example E. coli) can be transformed with amylin recombinant plasmid.

The transformed E. coli host cells may be cultured under appropriate conditions to express amylin or a amylin-like peptide.

It will therefore be seen that according to further aspects of the invention, there are provided:
- a DNA or RNA sequence coding for a peptide as described above:
- a vector (such as a plasmid) comprising such a DNA sequence; and
- a host cell (for example a bacterial cell or eukaryotic (eg yeast) cell) comprising such a vector and capable of expressing such a DNA sequence.

Amylin and other peptides and peptide preparations in accordance with the invention may be found to have clinical utility, such as vasodilator activity, which could be either general activity or be specific for pancreas or islet blood flow. According to a further aspect of the invention, there is provided amylin or a conservative substitution thereof or a sub-fragment thereof (as defined above) in accordance with the first aspect for use in human or veterinary medicine, for example in the preparation of a vasodilator.

Further preferred features are defined in the dependent claims.

For a better understanding of the present invention, and to show how it may be put into effect, reference will now be made by way of example to the following experimental work and the accompanying drawings in which:
FIGURE 1A shows the absorbence profile of HPLC gel filtration eluent in 6 M guanidine HCl/0.2 M sodium phosphate pH 7.5 of material derived from an amyloid containing diabetic pancreas. Amylin is present in the region shown by the bar;
FIGURE 1B, 1C and 1D show reverse phase HPLC absorbence profiles; and
FIGURE 2 show comparisons of the structure of DAP as against various homologous molecules.

### EXAMPLE 1

Pancreata from diabetics were obtained at autopsy after death and frozen at -20°C or less until extraction. Islet amyloid was detected in tissue fixed in 150mM NaCl/formalin 10% by light microscopy after haematoxylin and eosin staining, and confirmed after staining with alkaline Congo red by the demonstration of green birefringence by microscopy under polarized light.

Whole pancreata were homogenized in ice cold 150 mM NaCl 1:4 (w/v) and pelleted in a Sorvall RC-5B centrifuge using a GS-3 head at 10,000g for 30 minutes at 4°C. The fat and supernatant layers were then discarded and the process was repeated twice. Crude lyophilised collagenase (EC 3.4.24.3, Boehringerher-Mannheim UK Ltd - Produce 103586) was dissolved 1:100 (w/v) in buffer, 50mM Tris-HCl, 150mM sodium chloride, 3mM CaCl₂, (2% v/v) NONIDET-P40, pH 7.4, and purified in a Beckman LR 5B centrifuge, S.W. 40 Ti rotor at 18,000g for 2 hours. Aliquots of the crude pancreatic homogenate were heated to 70°C for 10 minutes and incubated with the collagenase supernate (1:10 w/v) for 20 hours at 37°C with continuous vigorous shaking. Aliquots were pelleted in siliconised microcentrifuge tubes for 10 minutes at 11,200g, the supernatent discarded and the procedure repeated twice with 10 volumes of 150mM NaCl and once with 10 volumes of distilled water. Staining of aliquots with alkaline Congo red revealed 20-50% of the residue material was particles of amyloid (average diameter 10 to 30 microns), which were not seen in any of the non-diabetic, amyloid-negative control pancreata (see Example 2). The solubility of the amyloid was assessed by shaking continuously in a variety of solvents for two days, with repelleting at 11,200g for 10 minutes, followed by microscopy after alkaline Congo red staining and protein analysis of the supernatants.

The amyloid was solubilised by ultra-sound (MSE Sonic Desintegrator, Model 150w, wavelength 8 microns, 20kHz) into 70% (v/v) formic acid at 1/4 (w/v). Ultra-sound was delivered in four 30 second bursts with cooling in a dry ice/ethanol bath for 15 seconds after each burst. The formic acid was immediately removed by rotary evacuation to near dryness in a Sorvall SPEED VAC (Sorvall UK Ltd) and the amyloid was re-solubilised in 6 M guanidine/0.2 M sodium phosphate pH 7.5 with constant shaking for one hour.

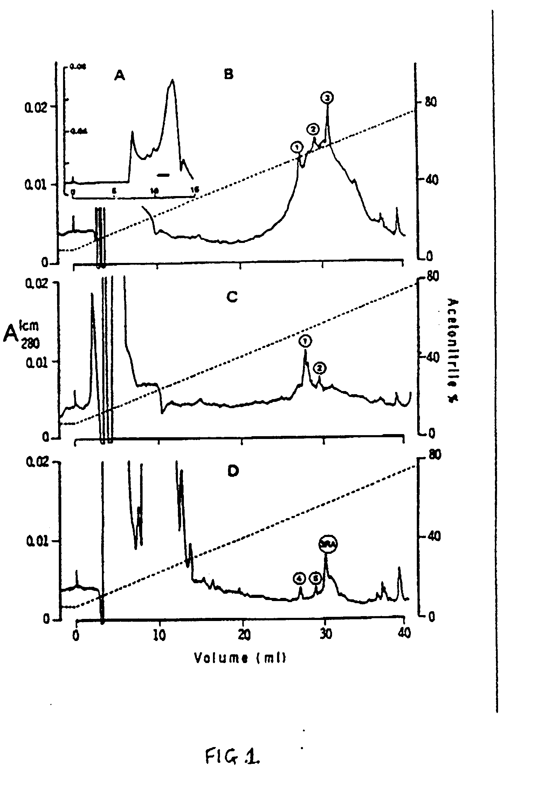

Initial separation was achieved by high performance liquid chromatography (HPLC) gel filtration chromatography on ZORBAX GF450 and GF250 columns (250 x 9.4mm DuPont (UK) Ltd) in series, in a Waters system with mobile phase 6 M guanidine/0.2 M sodium phosphate pH 7.5 and the runs were monitored at 280nm. The trace shown in Figure 1A shows the absorbance at 280nm.

Samples from the gel filtration system were injected directly onto a PARTISIL-10 ODS-3 reverse phase HPLC column (300 x 4mm Whatman Ltd). The stationary phase was hydrophobic. The mobile phase was 1% trifluoroacetic acid (TFA) with linear gradient elution by acetonitrile (5 to 80% over 45 minutes). Runs were again monitored at 280nm.

Figure 1B shows the trace. Peak 3 contains native DAP. Peaks 1 and 2 appear to be contaminants, which survived the initial HPLC gel filtration purification. The elevation of baseline absorbence seen in this Figure is produced by non-proteinaceous material, which may be lipid.

Quantitative protein determination and amino acid compositions were made using a Waters PICO-TAG amino acid analysis system (Cohen et al. American Laboratory August 1984, 48 and an Applied Biosystems 470A protein sequencer (Herrick et al. J. Biol. Chem. 256 (1981) 7990) using the O2CPTH cycle in the Version 2.0 software (Applied Biosystems Ltd.) Phenylthiohydatoin amino acid derivatives were identified by HPLC. The resulting sequence has been described above.

### EXAMPLE 2 (COMPARISON EXAMPLE)

The procedure of Example 1 was repeated, except that pancreata from non-diabetics were obtained after autopsy. After collagenase digestion and pelleting, Congo red staining did not reveal any of the spherical amyloid particles. However, if the procedure was nevertheless continued to the gel filtration HPLC step, the absorbence profile was similar with the diabetic, amyloid-containing samples. This absorbence profile could therefore not be used as a guide to the presence or absence of dissolved amyloid monomer. Reinjection of a sample from the gel filtration HPLC system directly onto a reverse phase HPLC column gave rise to a reverse phase HPLC trace as shown in Figure 1C. It can be seen that peaks corresponding to peaks 1 and 2 in Figure 1B are present, but there is no peak corresponding to peak 3 of Figure 1B. This peak was in fact found to be present in each of three diabetic pancreata extracted and in none of six amyloid-negative, non-diabetic control pancreata. It eluted at an acetonitrile concentration of 61.5%.

### EXAMPLE 3

For the purposes of further analysis and purification, the amylin obtained in Example 1 was subjected to reduction and alkylation in the following way. Tryptic cleavage (TACK-trypsin (Worthington UK)) of aliquots of peptide was for 3 hours at 37°C in 100mM ammonium bicarbonate buffer, enzyme:substrate ratio 1:100, with termination of the reaction by the addition of diisopropylfluorophosphate to 25mM. C-terminal sequencing with carboxypeptidase Y was performed in 0.2 M pyridine-acetic acid buffer pH 5.5 with termination of reaction and 100°C for 2 minutes. Reduction and radio S-carboxymethylation of cysteine residues were performed in 6 M guanidine/0.2 M tris pH 8.0/3mM sodium ethylene diamine tetraacetate (EDTA) by the addition, to 20mM dithiothreitol of approximately 1 nmole of purified peptide, shaking for 3 hours and subsequent addition of ¹⁴C-labelled iodoacetic acid (IAA) for 5 minutes, at 0°C in the dark, followed by freshly neutralized (with 4 M NaOH) non-radio labelled IAA to 40mM.

The thus-derivatised peptide was repurified on the same reverse phase HPLC system. It now eluted at the slightly earlier acetonitrile concentration of 64%, which is consistent with the introduction of the slightly more polar carboxymethyl group into the peptide. The absorbence profile can be seen in Figure 1D, where peak 3RA represents the derivatized peptide.

Two minor peptides, both of which had distinct amino acid analyses and both of which were amino-terminally blocked on sequence analysis, separated at this stage, can be seen as peaks 4 and 5 in Figure 1D. Amino acid analysis of the unpaired peak suggested the presence of a nearly-pure peptide, with a likely length of 37 amino acid residues. Its composition was distinctive, in that there was a ratio of 5 mole Asp or Asn to 1 mole Glu/Gln. This pattern was identical in the peptides purified from both of the two diabetic pancreases.

Amino acid sequence analysis gave the same results for peptides extracted from both of two diabetic pancreata. Sequence analysis was performed from both of two diabetic pancreata. Sequence analysis was also completed on native peptide after tryptic digestion, when the amino-terminal sequence was known and that after the Arg residue was determined by subtraction. From approximately 1000 p.mol of pure peptide, the initial Lys residue was present at the 400 p.mol level. Thereafter, on reduced and alkylated material, sequencing yields were at least 92%; however, when native material was sequenced, there was a large drop in yield at residue 2 and a second reduction at residue 7. This fact, plus the demonstration of normal yields after reduction/alkylation, suggests that the Cys residues at position 2 and 7 may form a disulphide bridge. C-terminal sequencing with CPase Y gave initially equivocal results, but also gave evidence that the C-terminal residue was Tyr.

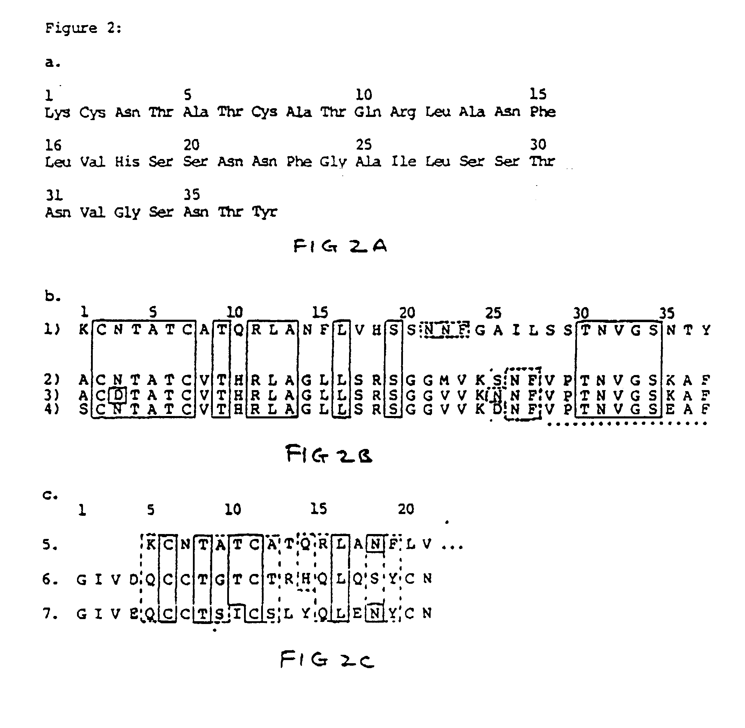

Figure 2A shows the structure of diabetes associated peptide, (DAP) or amylin.

Figure 2B shows a comparison of the primary structure sequence of native DAP (sequence 1) with the human calcitonin gene-related peptides CGRP-1 (sequence 3) and CGRP-2 (sequence 2) and rat CGRP-1 (sequence 4). The dotted boxes indicate areas of displaced homology.

Figure 2C shows a comparison of the primary structures of amylin (sequence 5) with the alpha-chains of guinea pig insulin (sequence 6) and human insulin (sequence 7). The number of the residues is as for insulin. A colon in a comparison sequence indicates identity and a fullstop indicates a conservative change. The dashed boxes represent areas of conservative amino acid substitution. Amino acid identity between peptides is indicated by boxes.

Assessment of homology by the ALIGN program and Mutation Data Matrix of Dayhoff et al. (Methods in Enzymology (1983) 91 524-545) gave the highly significant score of 8.31 for amylin versus Human CGRP-1, (Figure 2B, sequence 1 and sequence 3) confirming the close relationship between the two peptides. The score against the insulin alpha chain was not significant, largely because of the unmatched Cys residues at position 7 and 20 in the insulin alpha chains. Nevertheless, there is identity on three highly conserved residues in the insulin alpha chain (residues 6, 11 and 16) and a conservative change at a fourth position (Phe/Tyr at residue 19) (Figure 2C).

## Claims (Claims for the following Contracting State(s): AT, BE, CH, DE, FR, GB, IT, LU, LI, NL, SE)

1. A synthetic peptide having the amino acid sequence: 
    5    10   15   20   25   30   35


 having a disulphide bridge between the cysteine residues.

2. A synthetic peptide of claim 1 in which the 37th amino acid is tyrosine amide.

3. A synthetic peptide which is a conservative substitution of a peptide of either of claims 1 and 2 having a disulphide bridge between the cysteine residues.

4. A synthetic peptide which is a sub-fragment of the peptide of claim 1 including the sequence VGSNTY and having a disulfide bridge between the cysteine residues.

5. A peptide as claimed in any one of claims 1 to 4 which is at least about 90% pure.

6. A peptide as claimed in claim 5 which is at least about 95% pure.

7. A peptide as claimed in any preceding claim for use in human or animal therapy.

8. An isolated DNA or RNA sequence coding for a peptide sequence of claim 1.

9. An isolated DNA or RNA sequence coding according to claim 8 which is:

AAG TGC AAC ACA GCC ACC TGT GCC ACT CAA CGG CTG GCA AAT TTT
TTA GTT CAT TCC AGC AAC AAC TTT GGT GCC ATT CTC TCA TCT ACC
AAT GTG GGT TCC AAT ACC TAT.

10. A vector comprising a sequence as defined in either of claims 8 or 9.

11. A host cell comprising a vector as claimed in claim 10 and capable of expressing a sequence as defined in either of claims 8 or 9.

12. Use of a peptide as claimed in any of claims 1 to 7 in a process for preparing material for an immunoassay for the confirmation or prediction of Type 2 diabetes mellitus.

13. An antibody which is selective for a peptide as defined in any of claims 1 to 7.

14. An antibody as claimed in claim 13 which is labelled.

15. An antibody as claimed in claim 14 for use in directing a pharmaceutical agent or enzyme against amyloid in the pancreas of a diabetic patient.

16. An antibody as claimed in any of claims 13 to 15 which is monoclonal.

17. Hybridoma cells capable of producing a monoclonal antibody as claimed in claim 16.

18. Use of an antibody as claimed in claim 13 or as claimed in claim 16 as dependent on claim 13 for detecting amylin in vitro.

19. A peptide having the amino acid sequence of claim 1 obtainable from diabetic pancreata by a process comprising the steps of:
(a) homogenizing said pancreata;
(b) heating the material resulting from step (a);
(c) digesting the material from step (b) using collagenase;
(d) centrifuging the material from step (c) and collecting the resulting amyloid material;
(e) solubilizing the amyloid material from step (d) using 70% formic acid in conjunction with ultrasound and cooling;
(f) immediately removing the formic acid from the material of step (e);
(g) redissolving the material from step (f) and performing gel filtration chromatography using a mobile phase comprising aqueous guanidine; and
(h) subjecting the material from step (g) to reverse phase high performance liquid chromatography using a mobile phase comprising trifluoroacetic acid and elution by an acetonitrile gradient.

20. A peptide according to claim 19 having vasodilator activity.

21. A peptide according to either of claims 19 and 20 which is resistant to carboxy-peptidase Y.

## Claims (Claims for the following Contracting State(s): ES, GR)

1. A process for the preparation of a synthetic peptide in which there is formed a peptide having the sequence: 
5 10 15 20 25 30 35

 having a disulphide bridge between the cysteine residues.

2. A process according to claim 1 in which the 37th amino acid is formed as tyrosine amide.

3. A modification of the process according to claim 1 for the preparation of a synthetic peptide in which there is formed a sub-fragment of the peptide including the sequence VGSNTY and having a disulfide bridge between the cysteine residues.

4. A process according to claim 1 for the preparation of a peptide which is identical to the peptide having the said amino acid sequence of claim 1 obtainable from diabetic pancreata by a process comprising the steps of:
(a) homogenizing said pancreata;
(b) heating the material resulting from step (a);
(c) digesting the material from step (b) using collagenase;
(d) centrifuging the material from step (c) and collecting the resulting amyloid material;
(e) solubilizing the amyloid material from step (d) using 70% formic acid in conjunction with ultrasound and cooling;
(f) immediately removing the formic acid from the material of step (e);
(g) redissolving the material from step (f) and performing gel filtration chromatography using a mobile phase comprising aqueous guanidine; and
(h) subjecting the material from step (g) to reverse phase high performance liquid chromatography using a mobile phase comprising trifluoroacetic acid and elution by an acetonitrile gradient.

5. A process according to any one of claims 1 to 4 wherein the peptide formed has vasodilation activity.

6. A process according to any one of claims 1 to 4 wherein the peptide formed is resistant to carboxy-peptidase Y.

7. A process according to any one of claims 1 to 4 in which the peptide is purified to at least about 90% purity.

8. A process according to claim 7 in which the peptide is purified to at least 95% purity.

9. Use for the production of pharmaceutical or immunoassay material of a synthetic peptide having the amino acid sequence: 
5    10    15    20    25    30    35

 having a disulphide bridge between the cysteine residues.

10. Use according to claim 7 wherein the 37th amino acid is tyrosine amide.

11. Use for the production of pharmaceutical or immunoassay material of a peptide which is identical the peptide having the said amino acid sequence of claim 1 obtainable from diabetic pancreata by a process comprising the steps of:
(a) homogenizing said pancreata;
(b) heating the material resulting from step (a);
(c) digesting the material from step (b) using collagenase;
(d) centrifuging the material from step (c) and collecting the resulting amyloid material;
(e) solubilizing the amyloid material from step (d) using 70% formic acid in conjunction with ultrasound and cooling;
(f) immediately removing the formic acid from the material of step (e);
(g) redissolving the material from step (f) and performing gel filtration chromatography using a mobile phase comprising aqueous guanidine; and
(h) subjecting the material from step (g) to reverse phase high performance liquid chromatography using a mobile phase comprising trifluoroacetic acid and elution by an acetonitrile gradient.

12. Use according to claim 11 wherein the peptide formed is resistant to carboxy-peptidase Y.

13. Use according to any one of claims 7 to 12 for the production of pharmaceutical or immunoassay material of a peptide which is a conservative substitution of the peptide defined in said claims.

14. Use of a synthetic peptide or conservative substitution as defined in any one of claims 4 to 13 in a process for preparing a medicament for humans or animals for vasodilation.

15. Use of isolated DNA or RNA having the sequence: 
AAG TGC AAC ACA GCC ACC TGT GCC ACT CAA CGG CTG GCA AAT TTT
TTA GTT CAT TCC AGC AAC AAC TTT GGT GCC ATT CTC TCA TCT ACC
 AAT GTG GGT TCC AAT ACC TAT in the preparation of a peptide having a sequence as defined in either of claims 1 and 2.

16. Use according to claim 11 in which there is generated a vector containing said genetic sequence coding.

17. Use according to claim 16 in which said vector is comprised in a host cell and said genetic sequence coding is expressed by said host cell.

18. Use of a peptide or conservative substitution as defined in any of claims 4 to 12 in a process for preparing material for an immunoassay for the confirmation or prediction of Type 2 diabetes mellitus.

19. Use of a peptide or conservative substitution as defined in any of claims 4 to 12 as an antigen for the production of a selective antibody thereto.

20. Use as claimed in claim 19 wherein said antibody is labelled.

21. Use as claimed in claim 19 for the production of an antibody for directing a pharmaceutical agent or enzyme against amyloid in the pancreas of a diabetic patient.

22. Use as claimed in any of claims 19 to 21 for the production of an antibody which is monoclonal.

23. Use of hybridoma cells for the production of a monoclonal antibody as defined in claim 22.

24. Use of an antibody as defined in claim 18 or as defined in claim 23 for detecting amylin in vitro.

25. A method of preparing a peptide having the amino acid sequence of claims 1 or 2 from diabetic pancreata by a process comprising the steps of:
(a) homogenizing said pancreata;
(b) heating the material resulting from step (a);
(c) digesting the material from step (b) using collagenase;
(d) centrifuging the material from step (c) and collecting the resulting amyloid material;
(e) solubilizing the amyloid material from step (d) using 70% formic acid in conjunction with ultrasound and cooling;
(f) immediately removing the formic acid from the material of step (e);
(g) redissolving the material from step (f) and performing gel filtration chromatography using a mobile phase comprising aqueous guanidine; and
(h) subjecting the material from step (g) to reverse phase high performance liquid chromatography using a mobile phase comprising trifluoroacetic acid and elution by an acetonitrile gradient.

## Patentansprüche (Patentansprüche für folgende(n) Vertragsstaat(en): AT, BE, CH, DE, FR, GB, IT, LU, LI, NL, SE)

1. Synthetisches Peptid mit der Aminosäuresequenz: 
5         10        15        20        25        30        35

 das eine Disulfid-Brücke zwischen den Cystein-Resten aufweist.

2. Synthetisches Peptid nach Anspruch 1, bei welchem die 37. Aminosäure Tyrosinamid ist.

3. Synthetisches Peptid, das eine konservative Substitution eines Peptids nach Anspruch 1 und 2 ist und eine Disulfid-Brücke zwischen den Cystein-Resten aufweist.

4. Synthetisches Peptid, das ein Subfragment des Peptids nach Anspruch 1 ist, einschließend die Sequenz VGSNTY und mit einer Disulfid-Brücke zwischen den Cystein-Resten.

5. Peptid nach einem der Ansprüche 1 bis 4, das mindestens etwa 90 Prozent rein ist.

6. Peptid nach Anspruch 5, das mindestens etwa 95 Prozent rein ist.

7. Peptid nach einem der vorgenannten Ansprüche zur Verwendung in der Humantherapie oder der Veterinärtherapie.

8. Isolierte DNA- oder RNA-Sequenzcodierung für eine Peptidsequenz nach Anspruch 1.

9. Isolierte DNA- oder RNA-Sequenzcodierung nach Anspruch 8, die lautet:

AAG TGC AAC ACA GCC ACC TGT GCC ACT CAA CGG CTG GCA
AAT TTT TTA GTT CAT TCC AGC AAC AAC TTT GGT GCC ATT
CTC TCA TCT ACC AAT GTG GGT TCC AAT ACC TAT.

10. Vektor, umfassend eine Sequenz nach Anspruch 8 oder 9.

11. Wirtszelle, umfassend einen Vektor nach Anspruch 10, der zu einer Expression einer Sequenz nach einem der Ansprüche 8 oder 9 fähig ist.

12. Verwendung eines Peptids nach Anspruch 1 bis 7 in einem Verfahren zur Herstellung von Material für ein Immunoassay zur Feststellung oder Vorhersage von Diabetes Mellitus Typ II.

13. Antikörper, der für ein Peptid nach einem der Ansprüche 1 bis 7 selektiv ist.

14. Antikörper nach Anspruch 13, der markiert ist.

15. Antikörper nach Anspruch 14 zur Verwendung, um ein pharmazeutisches Mittel oder Enzym gegen Amyloid in dem Pankreas eines Diabetes-Patienten zu richten.

16. Antikörper nach einem der Ansprüche 13 bis 15, der monoklonal ist.

17. Hybridoma-Zellen, die einen monoklonalen Antikörper nach Anspruch 16 erzeugen können.

18. Verwendung eines Antikörpers nach Anspruch 13 oder nach dem von Anspruch 13 abhängigen Anspruch 16, zum Detektieren von Amylin in vitro.

19. Peptid mit der Aminosäuresequenz nach Anspruch 1, das aus diabetischem Pankreas nach einem Verfahren erhalten werden kann, umfassend die Schritte:
(a) Homogenisieren der Pankreas;
(b) Erhitzen des aus Schritt (a) resultierenden Materials;
(c) Digerieren des in Schritt (b) erhaltenen Materials unter Verwendung von Collagenase;
(d) Zentrifugieren des Materials von Schritt (c) und Aufnehmen des resultierenden Amyloid-Materials;
(e) Solubiliseren des Amyloid-Materials von Schritt (d) unter Verwendung von 70 %iger Ameisensäure in Verbindung mit Ultraschall und Kühlen;
(f) sofortiges Entfernen der Ameisensäure aus dem Material von Schritt (e);
(g) Wiederauflösen des Materials von Schritt (f) und Ausführen der Gelfiltrationschromatographie unter Verwendung einer mobilen Phase mit wässrigem Guanidin; und
(h) das Material von Schritt (g) einer Umkehrphasen-Hochleistungschromatographie unterziehen unter Verwendung einer mobilen Phase mit Trifluoressigsäure und Eluieren mit Hilfe eines Acetonitril-Gradienten.

20. Peptid nach Anspruch 19 mit Vasodilatator-Aktivität.

21. Peptid nach einem der Ansprüche 19 und 20, das gegenüber Carboxy-Peptase Y beständig ist.

## Patentansprüche (Patentansprüche für folgende(n) Vertragsstaat(en): ES, GR)

1. Verfahren zur Herstellung eines synthetischen Peptids, wobei ein Peptid erzeugt wird mit der mit der Sequenz 
5         10        15        20        25        30        35

 das eine Disulfid-Brücke zwischen den Cystein-Resten aufweist.

2. Verfahren nach Anspruch 1, bei welchem die 37. Aminosäure als Tyrosinamid erzeugt wird.

3. Modifikation des Verfahrens nach Anspruch 1 für die Herstellung eines synthetischen Peptids, bei welchem Verfahren ein Subfragment des Peptids erzeugt wird, einschließend die Sequenz VGSNTY und mit einer Disulfid-Brücke zwischen den Cystein-Resten.

4. Verfahrens nach Anspruch 1 für die Herstellung eines Peptids, das identisch ist mit dem Peptid, das die Aminosäuresequenz nach Anspruch 1 aufweist und aus diabetischem Pankreas nach einem Verfahren erhalten werden kann, umfassend die Schritte:
(a) Homogenisieren des Pankreas;
(b) Erhitzen des aus Schritt (a) resultierenden Materials;
(c) Digerieren des in Schritt (b) erhaltenen Materials unter Verwendung von Collagenase;
(d) Zentrifugieren des Materials von Schritt (c) und Aufnehmen des resultierenden Amyloid-Materials;
(e) Solubiliseren des Amyloid-Materials von Schritt (d) unter Verwendung von 70 %iger Ameisensäure in Verbindung mit Ultraschall und Kühlen;
(f) sofortiges Entfernen der Ameisensäure aus dem Material von Schritt (e);
(g) Wiederauflösen des Materials von Schritt (f) und Ausführen der Gelfiltrationschromatographie unter Verwendung einer mobilen Phase mit wässrigem Guanidin; und
(h) das Material von Schritt (g) einer Umkehrphasen-Hochleistungschromatographie unterziehen unter Verwendung einer mobilen Phase mit Trifluoressigsäure und Eluieren mit Hilfe eines Acetonitril-Gradienten.

5. Verfahren nach einem der Ansprüche 1 bis 4, bei welchem das erzeugte Peptid Vasodilatator-Aktivität aufweist.

6. Verfahren nach einem der Ansprüche 1 bis 4, bei welchem das erzeugte Peptid gegenüber Carboxy-Peptase Y beständig ist.

7. Verfahren nach einem der Ansprüche 1 bis 4, bei welchem das Peptid zu einer Reinheit von mindestens 90 Prozent gereinigt ist.

8. Verfahren nach Anspruch 7, bei welchem das Peptid zu einer Reinheit von mindestens 95 Prozent gereinigt ist.

9. Verwendung eines synthetischen Peptids für die Herstellung eines pharmazeutischen oder Immunoassay-Materials, welches synthetische Peptid die Aminosäuresequenz: 
5         10        15        20        25        30        35

 und eine Disulfid-Brücke zwischen den Cystein-Resten aufweist.

10. Verwendung nach Anspruch 7, wobei die 37. Aminosäure Tyrosinamid ist.

11. Verwendung eines Peptids für die Herstellung eines pharmazeutischen oder Immunoassay-Materials, welches Peptid identisch mit dem Peptid ist, das die Aminosäuresequenz nach Anspruch 1 aufweist und aus diabetischem Pankreas nach einem Verfahren erhalten werden kann, umfassend die Schritte:
(a) Homogenisieren des Pankreas;
(b) Erhitzen des aus Schritt (a) resultierenden Materials;
(c) Digerieren des in Schritt (b) erhaltenen Materials unter Verwendung von Collagenase;
(d) Zentrifugieren des Materials von Schritt (c) und Aufnehmen des resultierenden Amyloid-Materials;
(e) Solubiliseren des Amyloid-Materials von Schritt (d) unter Verwendung von 70 %iger Ameisensäure in Verbindung mit Ultraschall und Kühlen;
(f) sofortiges Entfernen der Ameisensäure aus dem Material von Schritt (e);
(g) Wiederauflösen des Materials von Schritt (f) und Ausführen der Gelfiltrationschromatographie unter Verwendung einer mobilen Phase mit wässrigem Guanidin; und
(h) das Material von Schritt (g) einer Umkehrphasen-Hochleistungschromatographie unterziehen unter Verwendung einer mobilen Phase mit Trifluoressigsäure und Eluieren mit Hilfe eines Acetonitril-Gradienten.

12. Verwendung nach Anspruch 11, wobei das erzeugte Peptid gegenüber Carboxy-Peptase Y beständig ist.

13. Verwendung eines Peptids nach einem der Ansprüche 7 bis 12 zur Herstellung eines pharmazeutischen oder Immunoassay-Materials, welches Peptid eine konservative Substitution eines Peptids nach einem der vorgenannten Ansprüche ist.

14. Verwendung eines synthetischen Peptids oder einer konservativen Substitution nach einem der Ansprüche 4 bis 13 in einem Verfahren zur Herstellung eines Medikaments zur Vasodilatation bei Mensch oder Tier.

15. Verwendung isolierter DNA oder RNA mit der Sequenz: 
AAG TGC AAC ACA GCC ACC TGT GCC ACT CAA CGG CTG GCA
AAT TTT TTA GTT CAT TCC AGC AAC AAC TTT GGT GCC ATT
CTC TCA TCT ACC AAT GTG GGT TCC AAT ACC TAT.
 in der Herstellung eines Peptids mit der in Anspruch 1 und Anspruch 2 festgelegten Sequenz.

16. Verwendung nach Anspruch 11, wobei ein Vektor erzeugt wird, der die genetische Sequenz-Codierung enthält.

17. Verwendung nach Anspruch 16, wobei der Vektor in einer Wirtszelle ist und die genetische Sequenz-Codierung durch diese Wirtszelle expressiert wird.

18. Verwendung eines synthetischen Peptids oder einer konservativen Substitution nach einem der Ansprüche 4 bis 12 in einem Verfahren zur Herstellung eines Materials für ein Immunoassay zur Feststellung oder Vorhersage von Diabetes Mellitus Typ II.

19. Verwendung eines synthetischen Peptids oder einer konservativen Substitution nach einem der Ansprüche 4 bis 12 als ein Antigen für die Herstellung von selektivem Antikörper dafür.

20. Verwendung nach Anspruch 19, wobei der Antikörper markiert ist.

21. Verwendung nach Anspruch 19 für die Herstellung eines Antikörpers, um ein pharmazeutisches Mittel oder Enzym gegen Amyloid in dem Pankreas eines Diabetes-Patienten zu richten.

22. Verwendung nach einem der Ansprüche 19 bis 21 für die Herstellung eines Antikörpers, der monoklonal ist.

23. Verwendung von Hybridoma-Zellen für die Herstellung eines monokolonalen Antikörpers nach Anspruch 22.

24. Verwendung eines Antikörpers nach Anspruch 18 oder nach Anspruch 23, um Amylin in vitro zu detektieren.

25. Verfahren zum Herstellen eines Peptids mit der Aminosäuresequenz nach Anspruch 1 oder 2 aus diabetischem Pankreas nach einem Verfahren, umfassend die Schritte:
(a) Homogenisieren der Pankreas;
(b) Erhitzen des aus Schritt (a) resultierenden Materials;
(c) Digerieren des in Schritt (b) erhaltenen Materials unter Verwendung von Collagenase;
(d) Zentrifugieren des Materials von Schritt (c) und Aufnehmen des resultierenden Amyloid-Materials;
(e) Solubiliseren des Amyloid-Materials von Schritt (d) unter Verwendung von 70 %iger Ameisensäure in Verbindung mit Ultraschall und Kühlen;
(f) sofortiges Entfernen der Ameisensäure aus dem Material von Schritt (e);
(g) Wiederauflösen des Materials von Schritt (f) und Ausführen der Gelfiltrationschromatographie unter Verwendung einer mobilen Phase mit wässrigem Guanidin; und
(h) das Material von Schritt (g) einer Umkehrphasen-Hochleistungschromatographie unterziehen unter Verwendung einer mobilen Phase mit Trifluoressigsäure und Eluieren mit Hilfe eines Acetonitril-Gradienten.

## Revendications (Revendications pour l'(les) Etat(s) contractant(s) suivant(s): AT, BE, CH, DE, FR, GB, IT, LU, LI, NL, SE)

1. Peptide synthétique présentant la séquence d'acides aminés 
5 10 15 20 25 30 35


 comportant un pont disulfure entre les radicaux de cystéine.

2. Peptide synthétique suivant la revendication 1, caractérisé en ce que le 37ème acide aminé est de l'amide de tyrosine.

3. Peptide synthétique, caractérisé en ce qu'il est une substitution conservatrice d'un peptide suivant l'une ou l'autre des revendications 1 et 2 comportant un pont disulfure entre les radicaux de cystéine.

4. Peptide synthétique, caractérisé en ce qu'il est un sous-fragment du peptide de la revendication 1 incluant la séquence VGSNTY et comportant un pont disulfure entre les radicaux de cystéine.

5. Peptide suivant l'une quelconque des revendications 1 à 4, caractérisé en ce qu'il est pur à au moins environ 90 %.

6. Peptide suivant la revendication 5, caractérisé en ce qu'il est pur à au moins environ 95 %.

7. Peptide suivant l'une quelconque des revendications précédentes, à mettre en oeuvre dans une thérapie humaine ou animale.

8. Séquence d'ADN ou d'ARN isolée codant pour une séquence peptidique suivant la revendication 1.

9. Séquence codante d'ADN ou d'ARN isolée suivant la revendication 8, qui est :

AAG TGC AAC ACA GCC ACC TGT GCC ACT CAA CGG CTG GCA AAT TTT
TTA GTT CAT TCC AGC AAC AAC TTT GGT GCC ATT CTC TCA TCT ACC
AAT GTG GGT TCC AAT ACC TAT.

10. Vecteur comprenant une séquence suivant l'une ou l'autre des revendications 8 et 9.

11. Cellule hôte comprenant un vecteur suivant la revendication 10 et capable d'exprimer une séquence suivant l'une ou l'autre des revendications 8 et 9.

12. Utilisation d'un peptide suivant l'une quelconque des revendications 1 à 7, dans un procédé de préparation de matière pour un essai immunologique en vue de la confirmation ou de la prédiction du diabète sucré de type 2.

13. Anticorps, caractérisé en ce qu'il est sélectif pour un peptide suivant l'une quelconque des revendications 1 à 7.

14. Anticorps suivant la revendication 13, caractérisé en ce qu'il est marqué.

15. Anticorps suivant la revendication 14, à mettre en oeuvre pour diriger une enzyme ou agent pharmaceutique contre un amyloïde dans le pancréas d'un patient diabétique.

16. Anticorps suivant l'une quelconque des revendications 13 à 15, caractérisé en ce qu'il est monoclonal.

17. Cellules d'hybridome capables de produire un anticorps monoclonal suivant la revendication 16.

18. Utilisation d'un anticorps suivant la revendication 13 ou suivant la revendication 16 en tant que revendication dépendant de la revendication 13, pour détecter de l'amyline in vitro.

19. Peptide présentant la séquence d'acides aminés de la revendication 1, qui peut être obtenu de pancréas de diabétiques par un procédé comprenant les étapes suivantes :
(a) une homogénéisation des pancréas,
(b) un chauffage de la matière résultant de l'étape (a),
(c) une digestion de la matière de l'étape (b) en utilisant de la collagénase,
(d) une centrifugation de la matière de l'étape (c) et une récolte de la matière amyloïde résultante,
(e) une solubilisation de la matière amyloïde de l'étape (d) en utilisant de l'acide formique à 70 % conjointement à des ultrasons et à un refroidissement,
(f) une élimination immédiate de l'acide formique à partir de la matière de l'étape (e),
(g) une redissolution de la matière de l'étape (f) et une réalisation d'une chromatographie par filtration sur gel en utilisant une phase mobile comprenant de la guanidine aqueuse, et
(h) une exposition de la matière de l'étape (g) à une chromatographie en phase liquide à haute performance et à phase inverse en utilisant une phase mobile comprenant de l'acide trifluoroacétique et une élution par un gradient d'acétonitrile.

20. Peptide suivant la revendication 19, présentant une activité vasodilatatrice.

21. Peptide suivant l'une ou l'autre des revendications 19 et 20, caractérisé en ce qu'il est résistant à de la carboxy-peptidase Y.

## Revendications (Revendications pour l'(les) Etat(s) contractant(s) suivant(s): ES, GR)

1. Procédé de préparation d'un peptide synthétique, dans lequel il est formé un peptide présentant la séquence : 
5 10 15 20 25 30 35

 comportant un pont disulfure entre les radicaux de cystéine.

2. Procédé suivant la revendication 1, dans lequel le 37ème acide aminé est formé d'amide de tyrosine.

3. Modification du procédé suivant la revendication 1 de préparation d'un peptide synthétique, dans laquelle il est formé un sous-fragment du peptide incluant la séquence VGSNTY et comportant un pont disulfure entre les radicaux de cystéine.

4. Procédé suivant la revendication 1 pour la préparation d'un peptide identique au peptide présentant la séquence d'acides aminés de la revendication 1, qui peut être obtenu à partir de pancréas de diabétiques par un procédé comprenant les étapes suivantes :
(a) une homogénéisation des pancréas,
(b) un chauffage de la matière résultant de l'étape (a),
(c) une digestion de la matière de l'étape (b) en utilisant de la collagénase,
(d) une centrifugation de la matière de l'étape (c) et une récolte de la matière amyloïde résultante,
(e) une solubilisation de la matière amyloïde de l'étape (d) en utilisant de l'acide formique à 70 % conjointement à des ultrasons et à un refroidissement,
(f) une élimination immédiate de l'acide formique à partir de la matière de l'étape (e),
(g) une redissolution de la matière de l'étape (f) et une réalisation d'une chromatographie par filtration sur gel en utilisant une phase mobile comprenant de la guanidine aqueuse, et
(h) une exposition de la matière de l'étape (g) à une chromatographie en phase liquide à haute performance et à phase inverse utilisant une phase mobile comprenant de l'acide trifluoroacétique et une élution par un gradient d'acétonitrile.

5. Procédé suivant l'une quelconque des revendications 1 à 4, caractérisé en ce que le peptide formé présente une activité vasodilatatrice.

6. Procédé suivant l'une quelconque des revendications 1 à 4, caractérisé en ce que le peptide formé est résistant à de la carboxy-peptidase Y.

7. Procédé suivant l'une quelconque des revendications 1 à 4, caractérisé en ce que le peptide est purifié à une pureté d'au moins environ 90 %.

8. Procédé suivant la revendication 7, caractérisé en ce que le peptide est purifié à une pureté d'au moins 95 %.

9. Utilisation, pour la production d'une matière pharmaceutique ou d'essai immunologique, d'un peptide synthétique présentant la séquence d'acides aminés 
5    10   15   20   25   30   35

 comportant un pont disulfure entre les radicaux de cystéine.

10. Utilisation suivant la revendication 9, dans laquelle le 37ème acide aminé est de l'amide de tyrosine.

11. Utilisation, pour la production d'une matière pharmaceutique ou d'essai immunologique, d'un peptide identique au peptide présentant la séquence d'acides aminés de la revendication 1, qui peut être obtenu à partir de pancréas de diabétiques par un procédé comprenant les étapes suivantes :
(a) une homogénéisation des pancréas,
(b) un chauffage de la matière résultant de l'étape (a),
(c) une digestion de la matière de l'étape (b) en utilisant de la collagénase,
(d) une centrifugation de la matière de l'étape (c) et une récolte de la matière amyloïde résultante,
(e) une solubilisation de la matière amyloïde de l'étape (d) en utilisant de l'acide formique à 70 % conjointement à des ultrasons et à un refroidissement,
(f) une élimination immédiate de l'acide formique à partir de la matière de l'étape (e),
(g) une redissolution de la matière de l'étape (f) et une réalisation d'une chromatographie par filtration sur gel en utilisant une phase mobile comprenant de la guanidine aqueuse, et
(h) une exposition de la matière de l'étape (g) à une chromatographie en phase liquide à haute performance et à phase inverse utilisant une phase mobile comprenant de l'acide trifluoroacétique et une élution par un gradient d'acétonitrile.

12. Utilisation suivant la revendication 11, caractérisée en ce que le peptide formé est résistant à de la carboxy-peptidase Y.

13. Utilisation suivant l'une quelconque des revendications 7 à 12, pour la production d'une matière pharmaceutique ou d'essai immunologique, d'un peptide qui est une substitution conservatrice du peptide défini dans les revendications susdites.

14. Utilisation d'un peptide synthétique ou d'une substitution conservatrice suivant l'une quelconque des revendications 4 à 13 dans un procédé de préparation d'un médicament relatif à la vasodilatation chez les êtres humains ou les animaux.

15. Utilisation d'ADN ou d'ARN isolé présentant la séquence: 
AAG TGC AAC ACA GCC ACC TGT GCC ACT CAA CGG CTG GCA AAT TTT
TTA GTT CAT TCC AGC AAC AAC TTT GGT GCC ATT CTC TCA TCT ACC
AAT GTG GGT TCC AAT ACC TAT
 dans la préparation d'un peptide présentant une séquence suivant l'une ou l'autre des revendications 1 et 2.

16. Utilisation suivant la revendication 11, caractérisée en ce qu'un vecteur contenant la séquence génétique codante y est engendré.

17. Utilisation suivant la revendication 16, caractérisé en ce que le vecteur est renfermé dans une cellule hôte et en ce que la séquence génétique codante est exprimée par la cellule hôte.

18. Utilisation d'un peptide ou d'une substitution conservatrice suivant l'une quelconque des revendications 4 à 12, dans un procédé de préparation d'une matière pour un essai immunologique destiné à la confirmation ou à la prédiction du diabète sucré de type 2.

19. Utilisation d'un peptide ou d'une substitution conservatrice suivant l'une quelconque des revendications 4 à 12, comme antigène pour la production d'un anticorps sélectif vis-à-vis de celui-ci.

20. Utilisation suivant la revendication 19, caractérisée en ce que l'anticorps est marqué.

21. Utilisation suivant la revendication 19, pour la production d'un anticorps destiné à diriger une enzyme ou agent pharmaceutique contre un amyloïde dans le pancréas d'un patient diabétique.

22. Utilisation suivant l'une quelconque des revendications 19 à 21, pour la production d'un anticorps qui est monoclonal.

23. Utilisation de cellules d'hybridome pour la production d'un anticorps monoclonal suivant la revendication 22.

24. Utilisation d'un anticorps suivant la revendication 18 ou suivant la revendication 23, pour détecter de l'amyline in vitro.

25. Procédé de préparation d'un peptide présentant la séquence d'acides aminés suivant l'une ou l'autre des revendications 1 et 2, à partir de pancréas de diabétiques par un procédé comprenant les étapes suivantes :
(a) une homogénéisation des pancréas,
(b) un chauffage de la matière résultant de l'étape (a),
(c) une digestion de la matière de l'étape (b) en utilisant de la collagénase,
(d) une centrifugation de la matière de l'étape (c) et une récolte de la matière amyloïde résultante,
(e) une solubilisation de la matière amyloïde de l'étape (d) en utilisant de l'acide formique à 70 % conjointement à des ultrasons et à un refroidissement,
(f) une élimination immédiate de l'acide formique à partir de la matière de l'étape (e),
(g) une redissolution de la matière de l'étape (f) et une réalisation d'une chromatographie par filtration sur gel en utilisant une phase mobile comprenant de la guanidine aqueuse, et
(h) une exposition de la matière de l'étape (g) à une chromatographie en phase liquide à haute performance et à phase inverse utilisant une phase mobile comprenant de l'acide trifluoroacétique et une élution par un gradient d'acétonitrile.
